(19) Europäisches Patentamt
European Patent Office
Office européen des brevets

(11) Publication number: **0 138 617 B2**

(12) **NEW EUROPEAN PATENT SPECIFICATION**

(45) Date of publication of the new patent specification: **19.10.94**

(51) Int. Cl.5: **C07C 4/18**, B01J 29/36, C07C 5/27

(21) Application number: **84307092.1**

(22) Date of filing: **16.10.84**

# (54) Processes for the hydrodealkylation and/or isomerization of alkylaromatic hydrocarbons.

(30) Priority: **17.10.83 US 542483**

(43) Date of publication of application: **24.04.85 Bulletin 85/17**

(45) Publication of the grant of the patent: **25.01.89 Bulletin 89/04**

(45) Mention of the opposition decision: **19.10.94 Bulletin 94/42**

(84) Designated Contracting States: **AT BE CH DE FR GB IT LI LU NL SE**

(56) References cited:
**EP-A- 0 042 754**
**EP-A- 0 055 529**
**EP-A- 0 109 962**
**DE-B- 1 224 289**
**GB-A- 1 444 481**
**GB-A- 2 033 358**
**US-A- 4 152 363**
**US-A- 4 163 028**
**US-A- 4 172 813**
**US-A- 4 218 573**
**US-A- 4 236 996**

(73) Proprietor: **AMOCO CORPORATION**
**200 East Randolph Drive**
**Chicago Illinois 60601 (US)**

(72) Inventor: **Kutz, Nancy Ann**
**1219 E. Willow**
**Wheaton Illinois 60187 (US)**

(74) Representative: **Ritter, Stephen David et al**
**Mathys & Squire**
**10 Fleet Street**
**London EC4Y 1AY (GB)**

**EP 0 138 617 B2**

## Description

Background of the Invention

This invention relates to a process to convert alkyl-substituted aromatic compounds using a catalyst formed from a crystalline aluminosilicate molecular sieve and more particularly relates to a method of isomerizing xylenes and converting ethylbenzene in which ethylbenzene is converted substantially by hydrodealkylation.

Typically, in commercial manufacture of para-xylene, a feedstream containing $C_8$ aromatics (p-xylene, o-xylene, m-xylene and ethylbenzene) is used. From such stream p-xylene is removed, typically by crystallization or absorption, and the remaining mixture is contacted with a catalyst which isomerizes o-xylene and m-xylene to a mixture containing approximately a thermodynamic concentration of p-xylene. The isomerized mixture is recycled to the p-xylene removal unit. Within the process are units which remove by-products including fuel gases (such as ethane), benzene, toluene and heavy aromatics such as diethylbenzenes. Because of the difficulty of removing ethylbenzene from xylenes by distillation due to closeness of boiling points, it is desirable to convert ethylbenzene to other aromatic species by hydrodeethylation, which mainly produces ethane and benzene, or disproportionation/transethylation, which mainly produces benzene, diethylbenzenes and dimethylethylbenzenes. A commercially useful catalyst for the isomerization of xylenes typically converts ethylbenzene.

It has been found that crystalline aluminosilicate molecular sieves formulated as catalysts including a matrix material predominantly convert ethylbenzene through a disproportionation/transalkylation mechanism. Depending on the economic value of individual by-products from a para-xylene manufacturing unit and the composition of the aromatics feedstream, sometimes it is advantageous to convert more ethylbenzene through a hydrodealkylation method than a disproportionation/transethylation method. A catalyst based on aluminosilicate molecular sieves which converts alkyl aromatics, such as ethylbenzene, by hydrodealkylation would be very advantageous in some cases.

US Patent 4,400,573 describes use of crystalline borosilicate molecular sieve-based catalyst on which has been placed a molybdenum compound for conversion of alkyl aromatic hydrocarbons by hydrodealkylation.

US-A-4,172,813 describes a conversion process for the selective hydrodealkylation and transalkylation of reformates from which $C_8$ aromatics and lighter components have been largely removed. The conversion process uses as catalyst a large pore crystalline aluminosilicate in the presence of a tungsten/molybdenum compound.

An improved process to convert alkyl aromatic hydrocarbons by hydrodealkylation has been developed.

According to the invention there is provided a process for converting alkyl aromatic hydrocarbons at a temperature of from 95 °C to 700 °F (371 °C) by hydrodealkylation which comprises contacting an alkyl aromatic hydrocarbon feed containing xylenes and ethylbenzene under conversion conditions with a catalyst, whereby to isomerise xylenes and convert ethylbenzene substantially by hydrodeethylation, characterised in that the catalyst comprises an intermediate pore crystalline aluminosilicate zeolite-based catalyst composition having an internal pore diameter of less than 6.0 Angstroms but greater than 4.5 Angstroms incorporated within a matrix material and on which has been placed a molybdenum compound or a tungsten compound.

As indicated, the method of the invention uses a specific class of modified aluminosilicate molecular sieve materials as catalysts which convert alkyl aromatic compounds by hydrodealkylation. Such catalyst compositions comprise a suitable aluminosilicate zeolite material on which has been placed a molybdenum containing compound. Generally these catalysts exhibit catalytic properties which favor conversion of alkyl aromatics by hydrodealkylation.

Conversion of an alkyl aromatic hydrocarbon by hydrodealkylation cleaves the alkyl group from the aromatic nucleus in the presence of a hydrogen source and usually yields an alkane and an aromatic. Thus, ethylbenzene is converted by hydrodealkylation to ethane and benzene.

The crystalline aluminosilicate zeolite materials suitable in the invention are recognized in the art as intermediate pore, that is, having internal pore diameter of less than 6.0 Angstroms but greater than 4.5 Angstroms. Examples of such intermediate pore zeolites include ferrierite, ZSM-5 and ZSM-11. Other examples of intermediate pore zeolites useful in this invention include heulandite, clinoptilolite and dachiardite.

Molecular sieves characterized as "ferrierite" by chemical composition and X-ray spectra are known as naturally occurring materials and as synthesized materials. For example, a conventional ferrierite sieve is produced by crystallizing a basic mixture of sodium aluminate and an oxide of silicon without the use of an

organic template compound. Such ferrierites are described in D. W. Breck, "Zeolite Molecular Sieves," John Wiley & Sons, 1974, incorporated by reference herein. U.S. Patent 4,000,248 discloses a method of producing a ferrierite molecular sieve using N-methyl pyridinium hydroxide as an organic template compound in the crystallization of the sieve. U.S. Patents 4,016,245, 4,107,95 and 4,046,859 disclose the formation of a ferrierite-like material using an organic template derived from ethylenediamine, pyrrolidine or butanediamine, or organometallic 2-(hydroxyalkyl)trialkylaluminum compounds. U.S. Patent 4,323,481 describes a preparation of ferrierite using 2,4-pentanedione as a structure-directing agent. Breck describes the zeolites identified as heulandite, clinoptilolite and dachiardite.

Aluminosilicate molecular sieves identified as ZSM-5 are described in U.S. Patents 3,702.886 and 4,139,600. Such aluminosilicates are prepared using organic templates such as tetraalkyl ammonium salts, primary alkyl amines and alkylene diamines as described in U.S. Patents 4,139,600 and 4,151,189. Aluminosilicate molecular sieve identified as ZSM-11 is described in U.S. Patent 3,709,979.

The $SiO_2/Al_2O_3$ ratio of the aluminosilicate molecular sieve identified as ZSM-5 has been listed variously as between 5 and 100, as in U.S. Patent 3,702,886, and as between 5 and 500, as in U.S. Patent 4,139,600. A typical range is between 35 and 100.

Molybdenum compounds suitable for incorporation into catalysts of this invention include molybdenum compounds which can be placed onto the molecular sieve compositions described in this invention. Generally, water-soluble molybdenum compounds are suitable for impregnation, although molybdenum carbonyl can be used in gas-phase exchange techniques. Specific examples of water-soluble molybdenum compounds include ammonium heptamolybdate, molybdenum hydroxide, molybdenum trioxide, molybdenum oxydibromide, molybdenum oxytetrachloride, molybdenum oxydichloride, molybdenum oxychloride acid, molybdenum dioxydiflouride, molybdic acid, molybdic arsenic acid, molybdic silicic acid and ammonium permolybdate. The preferred molybdenum compound useful in this invention is ammonium heptamolybdate $[(NH_3)_6Mo_7O_{24} \cdot 4H_2O]$).

The hydrocarbon conversion process in which this invention is useful is a process to isomerize a mixture of xylenes while converting ethylbenzene to other hydrocarbon products. In such a process a mixture containing xylenes, usually deficient in para-xylene, and ethylbenzene is contacted with a catalyst composition such as one based on a crystalline aluminosilicate incorporated in a matrix material and impregnated with a molybdenum compound. The mixed xylenes, predominantly ortho- and meta-xylenes, are isomerized to a mixture containing para-xylene. The isomerized mixture preferably approximates a thermodynamic equilibrium mixture of xylenes which contains 23.5 wt.% p-xylene, 23.8 wt.% o-xylene and 52.7 wt.% m-xylene. Simultaneously with the isomerization of xylenes, ethylbenzene is converted to other hydrocarbon products, such as benzene, ethane, diethylbenzenes and ethylxylenes.

In more detail, the process of this invention is useful for liquid or vapor phase isomerization of xylenes and particularly the isomerization of mixed xylenes to para-xylene products with concurrent conversion of ethylbenzene. Operating conditions for isomerization of a xylene feed and ethylbenzene conversion broadly comprise a temperature of 95 °C to 371 °C, a hydrogen-to-hydrocarbon mole ratio of 0.5 to 20, a weight hourly space velocity (WHSV) of 0.01 weight unit of feed per hour per weight unit of catalyst ($hr.^{-1}$) to 90 $hr.^{-1}$, and a pressure of 0 to 8.2 MPa (0 psig to 1000 psig). Advantageously, the conditions comprise a a hydrogen-to-hydrocarbon mole ratio of 1 to 12, a WHSV of 1 $hr.^{-1}$ to 20 $hr.^{-1}$, and a pressure of 0 to 4.1 MPa (0 psig to 500 psig). The preferred conditions for the isomerization of xylenes and ethylbenzene conversion comprise a hydrogen-to-hydrocarbon mole ratio of 2 to 8, a WHSV of 1 $hr.^{-1}$ to 10 $hr.^{-1}$, and a pressure of 0 to 2.5 MPa (0 psig to 300 psig). Typically, a feed to such process contains 75 to 85 wt.% xylenes, 10 to 15 wt.% ethylbenzene, 0.2 to 1.0 wt.% paraffins and naphthenes, and 0.5 to 5 wt.% $C_9$ + aromatics.

In the process using this invention, a substantial method of ethylbenzene conversion is believed to be by hydrodealkylation. In such method, ethylbenzene in the presence of hydrogen is converted to benzene and ethane as represented by:

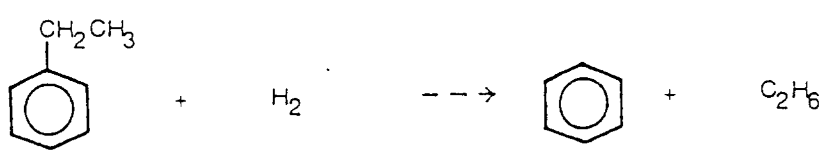

Ethylbenzene Hydrogen Benzene Ethane

In the process to convert ethylbenzene using crystalline aluminosilicate-based catalyst described herein in which a molybdenum compound is not present in the catalyst, it is believed that disproportionation/transalkylation is the predominant conversion method between two molecules of ethylbenzene or a molecule of ethylbenzene and a molecule of xylene as represented by:

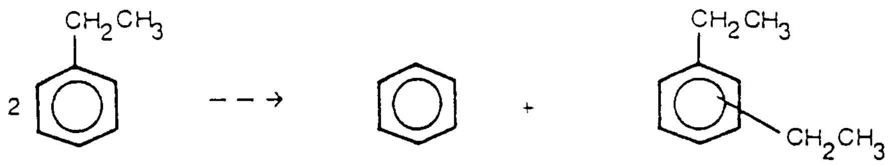

Ethylbenzene Benzene Diethylbenzene

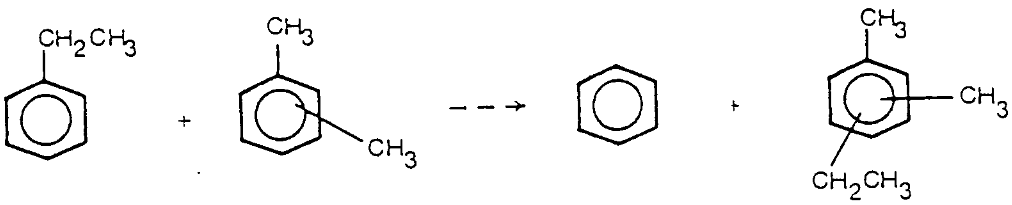

Ethylbenzene Xylene Benzene Ethylxylene

Catalysts based on crystalline aluminosilicate incorporated in a binder and impregnated with a molybdenum compound, used in a xylene isomerization/ethylbenzene conversion process, convert ethylbenzene mainly by a hydrodeethylation mechanism. Typically, about 40 to 90 percent of the ethylbenzene converted is converted by hydrodeethylation with the remainder converted by disproportionation/transethylation. The amount of hydrodeethylation activity in a specific aluminosilicate-based catalyst apparently depends, in part, on the amount and type of catalytically-active material placed onto the catalyst.

The isomerization catalyst system which is useful in this invention comprises a catalyst system based on intermediate pore crystalline aluminosilicate molecular sieve materials as described above.

Typically, the molybdenum-containing material can be placed onto the aluminosilicate structure by impregnation or other suitable contact means. Before placing a catalytically active compound on the aluminosilicate structure, the aluminosilicate may be in the hydrogen form which, typically, is produced by exchange one or more times with ammonium ion, typically using ammonium acetate, followed by drying and calcination as described above.

The original cation in the crystalline aluminosilicate, which usually is sodium ion, can be replaced all or in part by ion exchange with other cations including other metal ions and their amine complexes, alkylammonium ions, ammonium ions, hydrogen ions, and mixtures thereof. Ion exchange techniques are well-known in the art. Typically, an aqueous solution of a cationic species is exchanged one or more times at 25° to 100°C.

Typically, water soluble salts of molybdenum-containing materials are impregnated onto crystalline aluminosilicates used in this invention. Impregnation of a catalytically-active compound on the aluminosilicate or on a composition comprising the crystalline aluminosilicate suspended in and distributed throughout a matrix of a support material, such as a porous refractory inorganic oxide such as alumina, often results in a suitable catalytic composition. A combination of ion exchange and impregnation can be used. Presence of sodium ion in a composition usually is detrimental to catalytic activity.

The amount of molybdenum-containing material placed on the aluminosilicate catalyst composition can vary from less than 1 wt.% to about 30 wt.%, typically from 0.05 to 25 wt.%, depending on the process use intended. Preferably, for ethylbenzene conversion, 0.5 wt% to 10 wt% molybdenum is placed on the compositions of this invention. The optimum amount can be determined by routine experimentation.

Other catalytically-active species known in the art can be placed on the zeolite-based compositions of this invention by impregnation or exchange techniques. Examples of such other species include hydrogen and metal ions or compounds of Groups IB, IIB, IIIA, IVB, VB, VIB, VIIB and VIII, and of manganese, vanadium, chromium, uranium, and rare earth elements.

Although not preferable, for the purposes of this invention, tungsten is considered an equivalent to molybdenum.

The crystalline aluminosilicates useful in this invention may be incorporated as a pure material in a catalyst or adsorbent, or may be admixed with or incorporated within various binders or matrix materials depending upon the intended process use. The crystalline aluminosilicates can be combined with active or inactive materials, synthetic or naturally-occurring zeolites, as well as inorganic or organic materials which would be useful for binding the aluminosilicate. Well-known materials include silica, silica-alumna, alumina, alumina sols, hydrated aluminas, clays such as bentonite or kaolin, or other binders well-known in the art. Typically, the aluminosilicate is incorporated within a matrix material by blending with a sol of the matrix material and gelling the resulting mixture. Also, solid particles of the aluminosilicate and matrix material can be physically admixed. Typically, such aluminosilicate compositions can be pelletized or extruded into useful shapes. The crystalline aluminosilicate content can vary anywhere from a few up to 100 wt.% of the total composition. Catalytic compositions can contain 0.01 wt.% to 100 wt.% crystalline aluminosilicate material and preferably contain 2 wt.% to 65 wt.% of such material.

Catalytic compositions comprising the crystalline aluminosilicate material of this invention and a suitable matrix material can be formed by adding a finely-divided crystalline aluminosilicate and a molybdenum-containing compound to an aqueous sol or gel of the matrix material. The resulting mixture is thoroughly blended and gelled typically by adding a material such as ammonium hydroxide. The resulting gel can be dried and calcined to form a composition in which the crystalline aluminosilicate and molybdenum-containing metal compound are distributed through the matrix material.

Example I - Comparative Run A

A sample of HZSM-5 aluminosilicate zeolite containing 1.88 wt.% alumina was prepared according to U.S. Patent 3,702,866.

The sample of ZSM-5 crystalline aluminosilicate zeolite was prepared by dissolving 15.02 grams of sodium aluminate and 30.25 grams of sodium hydroxide in 1000 grams of distilled water followed by 62.02 grams of organic template. To this solution, 452.1 grams of Ludox HS-40 were added with vigorous stirring continuing for about 15 minutes after addition. The resulting curdy, gelatinous mixture was placed in a stirred, sealed crystallization vessel to crystallize for three days. The resulting crystalline material was recovered by filtration, washed thoroughly with distilled water, and dried in a forced draft oven at 165 °C for 4 hours. The dried material was program calcined with a program consisting of (a) a linear temperature rise of less than or equal to 100 °C per hour from 165 °C to 540 °C, (b) holding at 540 °C for 12 hours, and (c) decreasing the temperature at a maximum of 100 °C per hour from 540 °C to 120 °C. The sample was analyzed by X-ray diffraction spectroscopy and elemental analysis.

Portions of the zeolite so prepared were each exchanged with two times their weight of ammonium acetate in one liter of distilled water at 95 °C for two hours. The sieve then was filtered, washed with approximately 200 milliliters of distilled water, and filter dried. This procedure was repeated with a second exchange receiving a wash of about two liters of distilled water. The washed sieve was dried at 165 °C for approximately 3 hours. The dried sieve was program calcined with a program consisting of (a) a linear temperature rise of less than or equal to 100 °C per hour from 165 °C to 540 °C, (b) holding at 540 °C for 12 hours, and (c) decreasing the temperature at a maximum of 100 °C per hour from 540 °C to 120 °C. The catalyst was prepared by dispersing the above calcined and exchanged zeolite in PHF-alumina which is initially an acetic acid stabilized alumina hydrosol containing about 9% solids. To 40.03 grams of calcined and exchanged sieve was added 65.81 grams of distilled water to fill the sieve pores with water. The wet sieve was then added and thoroughly mixed with 607.6 grams of alumina hydrosol. The mixture was gelled (solidified) with the addition of 60 milliliters of concentrated ammonium hydroxide. The resulting gel was dried for 4 hours in a forced air oven at 165 °C and then program calcined to 540 °C with the program as described above, except that the 540 °C temperature was maintained for 4 hours.

A portion of this material (40.02 grams) was impregnated with 102.22 grams of an aqueous ammonium heptamolybdate solution (0.02 M) to give 3 wt.% molybdenum on the catalyst material. The impregnated catalyst was recalcined with the above-described 4-hour 540 °C program calcination. The calcined solid was crushed and sized to 18 to 40 mesh (U.S. Sieve Series).

A sample of 5.00 grams of HZSM-5 crystalline aluminosilicate-based catalyst prepared above was placed in a 0.5-inch inside diameter tubular reactor and pretreated with hydrogen at 0.755 SCF/hr at 319 °C and 180 psig for 2 hours; $C_8$ aromatic feed then was introduced in the reactor at about 0.75 grams/minute on a once-through basis with no recycle. Liquid effluent was analyzed by gas chromatography. Results from testing a molybdenum-impregnated sample (Example I) and a non-impregnated sample (Comparative Run A) are shown in Table I. The data show that the catalyst of Example I shows substantial ethylbenzene conversion by hydrodeethylation in contrast to the non-impregnated sample.

The amount of ethylbenzene converted by hydrodeethylation was calculated based on the following assumed transalkylation pathways:

Ethylbenzene + Xylenes $\longrightarrow$ Dimethylethylbenzenes + Benzene
(transethylation)

Ethylbenzene + Xylenes $\longrightarrow$ Ethyltoluene + Toluene
(transmethylation)

2 Ethylbenzene $\longrightarrow$ Diethylbenzenes + Benzene
(disproportionation)

Based on these pathways, the amount of ethylbenzene converted by hydrodeethylation in percent equals the moles of ethylbenzene reacted minus the sum of the moles of ethylbenzene converted by such transalkylation pathways, all divided by the moles of ethylbenzene reacted and multiplied by 100.

TABLE I

| ZSM-5 | Comparative Run A | | Example I | |
|---|---|---|---|---|
| **Conditions** | | | | |
| Molybdenum (wt.%) | 0 | | 3 | |
| Temperature (°C) | 319 | | 319 | |
| Pressure MPa (psig) | 3.5 (180) | | 3.5 (180) | |
| Hydrogen/Hydrocarbon (molar ratio) | 2.03 | | 1.98 | |
| Space Velocity (WHSV) ($hr^{-1}$) | 9.0 | | 9.0 | |
| **Components (wt.%)** | Feed | | Feed | |
| Paraffins & Naphthenes | 1.38 | 1.42 | 1.38 | 1.43 |
| Benzene | 0.0 | 1.65 | 0.0 | 1.90 |
| Toluene | 1.02 | 3.27 | 1.02 | 2.96 |
| Ethylbenzene | 15.55 | 11.62 | 15.52 | 11.98 |
| p-Xylene | 8.16 | 17.36 | 8.14 | 17.68 |
| m-Xylene | 48.15 | 39.53 | 48.11 | 39.98 |
| o-Xylene | 20.29 | 16.33 | 20.31 | 16.35 |
| Ethyltoluenes | 1.22 | 2.15 | 1.23 | 1.74 |
| Trimethylbenzenes | 0.51 | 1.06 | 0.52 | 1.03 |
| Diethylbenzenes | 1.66 | 2.56 | 1.69 | 2.17 |
| Dimethylethylbenzenes | 1.95 | 2.91 | 1.97 | 2.60 |
| Tetramethylbenzenes | 0.12 | 0.16 | 0.11 | 0.19 |
| **Results** | | | | |
| Ethylbenzene Conversion (%) | 25.2 | | 22.9 | |
| p-Xylene Approach to Equilibrium (%) | 99.1 | | 100.6 | |
| Ethylbenzene Conversion by | | | | |
| Hydrodeethylation (%) | 23.7 | | 51.7 | |
| Disproportionation (%) | 36.1 | | 21.4 | |
| Transmethylation (%) | 20.9 | | 12.8 | |
| Transethylation (%) | 19.3 | | 14.1 | |

Example II - Comparative Run B

A sample of ferrierite was prepared by the method described in U.S. Patent 4,323,481 using 2,4-pentanedione as the organic structure-directing agent.

A 25.02-gram portion of ferrierite aluminosilicate zeolite was impregnated with ammonium heptamolybdate as described in Example I. Samples of impregnated (Example II) and non-impregnated material (Comparative Run B) were tested as described in Example I. Results presented in Table II show that the molybdenum-impregnated catalyst converted ethylbenzene substantially by hydrodeethylation in contrast to Comparative Run B.

TABLE II

| Ferrierite | Comparative Run B | | Example II | |
|---|---|---|---|---|
| **Conditions** | | | | |
| Molybdenum (wt.%) | 0 | | 3 | |
| Temperature (°C) | 359 | | 360 | |
| Pressure MPa (psig) | 3.5 (180) | | 3.5 (180) | |
| Hydrogen/Hydrocarbon (molar ratio) | 2.04 | | 2.04 | |
| Space Velocity (WHSV) ($hr^{-1}$) | 6.06 | | 6.01 | |
| **Components (wt.%)** | Feed | | Feed | |
| Paraffins & Naphthenes | 1.40 | 1.37 | 1.45 | 1.39 |
| Benzene | 0.0 | 1.80 | 0.0 | 2.55 |
| Toluene | 1.03 | 2.01 | 1.05 | 2.04 |
| Ethylbenzene | 15.54 | 12.19 | 15.56 | 12.45 |
| p-Xylene | 8.16 | 17.56 | 8.16 | 18.02 |
| m-Xylene | 48.19 | 39.90 | 48.19 | 40.66 |
| o-Xylene | 20.28 | 17.62 | 20.25 | 17.88 |
| Ethyltoluenes | 1.22 | 1.37 | 1.20 | 0.96 |
| Trimethylbenzenes | 0.50 | 0.66 | 0.50 | 0.70 |
| Diethylbenzenes | 1.66 | 2.66 | 1.64 | 1.30 |
| Dimethylethylbenzenes | 1.94 | 2.77 | 1.91 | 2.01 |
| Tetramethylbenzenes | 0.07 | 0.09 | 0.09 | 0.04 |
| **Results** | | | | |
| Ethylbenzene Conversion (%) | 21.6 | | 20.0 | |
| p-Xylene Approach to Equilibrium (%) | 97.9 | | 99.0 | |
| Ethylbenzene Conversion by | | | | |
| Hydrodeethylation (%) | 29.4 | | 97.3 | |
| Disproportionation (%) | 47.0 | | 0.0 | |
| Transmethylation (%) | 4.1 | | 0.0 | |
| Transethylation (%) | 19.4 | | 2.7 | |

Comparative Runs C-F

Conversion tests, performed in a manner described in Example I, were conducted using samples of mordenite (Zeolon H) and Zeolite Y both with and without molybdenum impregnation. Both mordenite and Zeolite Y are considered large-pore zeolites. Results presented in Tables III and IV show that there is substantially no effect of molybdenum impregnation in conversion/selectivity tests using these large-pore zeolites.

TABLE III

| Mordenite | Feed | Comparative Run C | Comparative Run D |
|---|---|---|---|
| Conditions | | | |
| Molybdenum (wt.%) | | 0 | 3 |
| Temperature (°C) | | 359 | 360 |
| Pressure MPa (psig) | | 3.5 (180) | 3.5 (180) |
| Hydrogen/Hydrocarbon (molar ratio) | | 1.98 | 2.00 |
| Space Velocity (WHSV) ($hr^{-1}$) | | 5.98 | 6.02 |
| Components (wt.%) | Feed | | |
| Paraffins & Naphthenes | 1.45 | 1.28 | 1.31 |
| Benzene | 0.0 | 0.51 | 1.43 |
| Toluene | 1.04 | 1.89 | 5.43 |
| Ethylbenzene | 15.55 | 14.69 | 12.16 |
| p-Xylene | 8.16 | 11.56 | 15.87 |
| m-Xylene | 18.19 | 44.07 | 36.31 |
| o-Xylene | 20.26 | 19.07 | 15.04 |
| Ethyltoluenes | 1.21 | 1.50 | 2.84 |
| Trimethylbenzenes | 0.50 | 1.24 | 4.25 |
| Diethylbenzenes | 1.65 | 1.74 | 1.88 |
| Dimethylethylbenzenes | 1.92 | 2.33 | 3.28 |
| Tetramethylbenzenes | 0.07 | 0.11 | 0.23 |
| Results | | | |
| Ethylbenzene Conversion (%) | | 5.52 | 21.8 |
| p-Xylene Approach to Equilibrium (%) | | 37.0 | 99.5 |
| Ethylbenzene Conversion by | | | |
| Hydrodeethylation (%) | | 14.5 | 15.1 |
| Disproportionation (%) | | 17.9 | 10.8 |
| Transmethylation (%) | | 29.9 | 42.5 |
| Transethylation (%) | | 37.8 | 31.6 |

TABLE IV

| Zeolite Y | | Comparative Run E | Comparative Run F |
|---|---|---|---|
| **Conditions** | | | |
| Molybdenum (wt.%) | | 0 | 3 |
| Temperature (°C) | | 372 | 372 |
| Pressure MPa (psig) | | 3.5 (180) | 3.5 (180) |
| Hydrogen/Hydrocarbon (molar ratio) | | 1.96 | 2.01 |
| Space Velocity (WHSV) ($hr^{-1}$) | | 6.08 | 6.06 |
| **Components (wt.%)** | Feed | | |
| Paraffins & Naphthenes | 1.45 | 1.40 | 2.10 |
| Benzene | 0.0 | 1.12 | 1.25 |
| Toluene | 1.04 | 4.89 | 5.85 |
| Ethylbenzene | 15.55 | 13.59 | 13.35 |
| p-Xylene | 8.17 | 11.54 | 11.94 |
| m-Xylene | 48.19 | 38.88 | 36.73 |
| o-Xylene | 20.26 | 16.72 | 15.72 |
| Ethyltoluenes | 1.21 | 2.11 | 2.25 |
| Trimethylbenzenes | 0.50 | 4.67 | 5.74 |
| Diethylbenzenes | 1.64 | 1.50 | 1.33 |
| Dimethylethylbenzenes | 1.91 | 3.26 | 3.36 |
| Tetramethylbenzenes | 0.08 | 0.32 | 0.37 |
| **Results** | | | |
| Ethylbenzene Conversion (%) | | 12.6 | 14.2 |
| p-Xylene Approach to Equilibrium (%) | | 50.4 | 60.8 |
| Ethylbenzene Conversion by | | | |
| Hydrodeethylation (%) | | 4.7 | 5.8 |
| Disproportionation (%) | | 0.0 | 0.0 |
| Transmethylation (%) | | 40.7 | 42.0 |
| Transethylation (%) | | 54.6 | 52.2 |

## Claims

1. A process for converting alkyl aromatic hydrocarbons at a temperature of from 95 °C to 700 °F (371 °C) by hydrodealkylation which comprises contacting an alkyl aromatic hydrocarbon feed containing xylenes and ethylbenzene under conversion conditions with a catalyst, whereby to isomerise xylenes

and convert ethylbenzene substantially by hydrodeethylation, characterised in that the catalyst comprises an intermediate pore crystalline aluminosilicate zeolite-based catalyst composition having an internal pore diameter of less than 6.0 Angstroms but greater than 4.5 Angstroms incorporated within a matrix material and on which has been placed a molybdenum compound or a tungsten compound.

2. A process according to Claim 1 wherein the catalyst comprises a molybdenum compound.

3. A process according to Claim 1 or Claim 2 wherein the molybdenum compound is ammonium heptamolybdate or molybdenum carbonyl.

4. A process according to any preceding claim wherein 0.05 wt% to 25 wt% molybdenum-compound has been placed on the zeolite-based catalyst composition.

5. A process according to Claim 4 wherein 0.5 wt% to 10 wt% molybdenum has been placed on the zeolite-based catalyst composition.

6. A process according to Claim 1 wherein the catalyst comprises a tungsten compound.

7. A process according to any preceding claim wherein the aluminosilicate zeolite-based catalyst is incorporated within an alumina matrix.

8. A process according to any preceding claim wherein the aluminosilicate zeolite is ZSM-5.

9. A process according to any of Claims 1 to 7 wherein the aluminosilicate zeolite is ferrierite.

10. A process according to any preceding claim wherein the conversion conditions comprise a temperature of 95° to 371°C, a hydrogen-to-hydrocarbon molar ratio of 0.5 to 20, a weight hourly space velocity of 0.01 to 90 $hr^{-1}$, and a pressure of 0 to 8.2 MPa (0-1000 psig).

## Patentansprüche

1. Verfahren zur Umwandlung alkylsubstituierter aromatischer Kohlenwasserstoffe bei einer Temperatur von 95°C bis 700°F (371°C) durch Hydrodealkylierung, indem eine Beschickung aus dem jeweiligen alkylsubstituierten aromatischen Kohlenwasserstoff, welche Xylole und Ethylbenzol enthält, unter Umwandlungsbedingungen mit einem Katalysator behandelt wird, wobei die Xylole isomerisiert werden und das Ethylbenzol im wesentlichen durch Hydrodeethylierung umgewandelt wird, dadurch gekennzeichnet, daß der verwendete Katalysator eine Katalysatorzusammensetzung auf Basis eines sogenannten intermediärporigen kristallinen Aluminosilicat-Zeoliths ist, der einen inneren Porendurchmesser von weniger als 6,0 Angström, jedoch mehr als 4,5 Angström aufweist und in ein Matrixmaterial eingebaut ist, und auf dem eine Molybdänverbindung oder Wolframverbindung abgeschieden ist.

2. Verfahren nach Anspruch 1, dadurch gekennzeichnet, daß der Katalysator eine Molybdänverbindung enthält.

3. Verfahren nach Anspruch 1 oder 2, dadurch gekennzeichnet, daß die Molybdänverbindung Ammoniumheptamolybdat oder Molybdäncarbonyl ist.

4. Verfahren nach irgendeinem der vorhergehenden Ansprüche, dadurch gekennzeichnet, daß die Katalysatorzusammensetzung auf Basis eines Zeoliths 0,05 bis 25 Gewichtsprozent Molybdänverbindung enthält.

5. Verfahren nach Anspruch 4, dadurch gekennzeichnet, daß die Katalysatorzusammensetzung auf Basis eines Zeoliths 0,5 bis 10 Gewichtsprozent Molybdän enthält.

6. Verfahren nach Anspruch 1, dadurch gekennzeichnet, daß der Katalysator eine Wolframverbindung enthält.

7. Verfahren nach irgendeinem der vorhergehenden Ansprüche, dadurch gekennzeichnet, daß der Katalysator auf Basis eines Aluminosilicat-Zeoliths in einer Matrix aus Aluminiumoxid eingeschlossen ist.

8. Verfahren nach irgendeinem der vorhergehenden Ansprüche, dadurch gekennzeichnet, daß der Aluminosilicat-Zeolith ZSM-5 ist.

9. Verfahren nach irgendeinem der Ansprüche 1 bis 8, dadurch gekennzeichnet, daß der Aluminosilicat-Zeolith ein Ferrierith ist.

10. Verfahren nach irgendeinem der vorhergehenden Ansprüche, dadurch gekennzeichnet, daß die Umwandlung bei einer Temperatur von 95 bis 371 °C, unter einem Molverhältnis von Wasserstoff zu Kohlenwasserstoff von 0,5 bis 20, unter einer auf das Gewicht bezogenen stündlichen Raumgeschwindigkeit von 0,01 bis 90 $h^{-1}$ und bei einem Druck von 0 bis 8,2 MPa (0-1000 psig) durchgeführt wird.

## Revendications

1. Procédé pour convertir des hydrocarbures alcoylaromatiques à une température allant de 95 °C jusqu'à 700 °F (371 °C) par hydrodésalcoylation, qui comprend la mise en contact d'une charge hydrocarbonée alcoylaromatique contenant des xylènes et de l'éthylbenzène dans des conditions de conversion avec un catalyseur, permettant d'isomériser les xylènes et de convertir l'éthylbenzène sensiblement par hydrodéséthylation, procédé caractérisé en ce que le catalyseur comprend une composition catalytique à base de zéolite de type aluminosilicate cristallin à pores intermédiaires, ayant un diamètre des pores internes inférieur à 6,0 angströms, mais supérieur à 4,5 angströms, incorporée à une matrice et sur laquelle on a placé un composé de molybdène ou un composé de tungstène.

2. Procédé selon la revendication 1, dans lequel le catalyseur comprend un composé de molybdène.

3. Procédé selon la revendication 1 ou la revendication 2, dans lequel le composé de molybdène est l'heptamolybdate d'ammonium ou le molybdène-carbonyle.

4. Procédé selon l'une quelconque des revendications précédentes, dans lequel 0,05 en poids à 25% en poids de composé de molybdène ont été placés sur la composition catalytique à base de zéolite.

5. Procédé selon la revendication 4, dans lequel 0,5% à 10% en poids de molybdène ont été placés sur la composItion catalytique à base de zéolite.

6. Procédé selon la revendication 1, dans lequel le catalyseur comprend un composé de tungstène.

7. Procédé selon l'une quelconque des revendications précédentes, dans lequel le catalyseur à base de zéolite de type aluminosilicate est incorporé dans une matrice d'alumine.

8. Procédé selon l'une quelconque des revendications précédentes, dans lequel la zéolite de type aluminosilicate est la ZSM-5.

9. Procédé selon l'une quelconque des revendications 1 à 7, dans lequel la zéolite de type aluminosilicate est la ferriérite.

10. Procédé selon l'une quelconque de revendications précédentes, dans lequel les conditions de conversion comprennent une température de 95 à 371 °C, un rapport molaire hydrogène/hydrocarbure de 0,5 à 20, une vitesse spatiale pondérale horaire de 0,01 à 90 $h^{-1}$, et une pression de 0 à 8,2 MPa (0-1000 psig).